(19) Europäisches Patentamt
European Patent Office
Office européen des brevets

(11) EP 1 309 690 B1

(12) **EUROPEAN PATENT SPECIFICATION**

(45) Date of publication and mention of the grant of the patent:
**07.06.2006 Bulletin 2006/23**

(21) Application number: **01976072.7**

(22) Date of filing: **13.08.2001**

(51) Int Cl.:
*C12N 15/12* (2006.01) *C12N 15/55* (2006.01)
*C07K 14/47* (2006.01) *C12N 9/16* (2006.01)
*C07K 16/40* (2006.01) *C07K 16/18* (2006.01)
*G01N 33/573* (2006.01) *G01N 33/68* (2006.01)

(86) International application number:
**PCT/EP2001/009326**

(87) International publication number:
**WO 2002/016561 (28.02.2002 Gazette 2002/09)**

(54) **MFQ-111, A HUMAN GTPASE LIKE PROTEIN**

MFQ-111, EIN MENSCHLICHES GTPASE-ÄHNLICHERS PROTEIN

MFQ-111, PROTEINE DE TYPE GTPASE HUMAINE

(84) Designated Contracting States:
**AT BE CH CY DE DK ES FI FR GB GR IE IT LI LU MC NL PT SE TR**

(30) Priority: **18.08.2000 EP 00117781**

(43) Date of publication of application:
**14.05.2003 Bulletin 2003/20**

(73) Proprietor: **Merck Patent GmbH**
**64293 Darmstadt (DE)**

(72) Inventors:
- **MASA ALVAREZ, Marc**
**E-08020 Barcelona (ES)**
- **RODES GUBERN, Blanca**
**E-08849 Sant Climent de Llobregat (ES)**
- **MESSEGUER PEYPOCH, Ramon**
**E-08330 Premia de Mar (ES)**
- **ROSELL VIVES, Elisabet**
**E-08008 Barcelona (ES)**
- **PIULATS XANCO, Jaume**
**E-08030 Barcelona (ES)**

(56) References cited:
**WO-A-00/58473** **WO-A-01/57275**
**WO-A-02/04510** **WO-A-95/18226**

- **DATABASE GENESEQ [Online] " Human brain expressed single exon probe SEQ ID NO: 8102." Database accession no. AAK08111 XP002202339**
- **DATABASE EMBL [Online] 2 March 2000 (2000-03-02) "Human DNA sequence from clone RP11-235C23 on chromosome 9q31.1-31.3. Contains a pseudogene similar to KIAA1272, KIAA0884 and rat Tulip 1 and 2, the FCMD gene for Fukuyama type congenital muscular dystrophy protein, the TAL2 gene for T-cell acute lymphocytic leukemia 2 protein, ESTs, STSs, GSSs......" Database accession no. AL158070 XP002202340**
- **DATABASE EMBL [Online] 20 May 1999 (1999-05-20) " Homo sapiens mRNA; cDNA DKFZp566D133 (from clone DKFZp566D133); partial cds" Database accession no. AL050050 XP002202341 -& DATABASE SWISSPROT [Online] 1 November 1999 (1999-11-01) "HYPOTHETICAL 43.9 KDA PROTEIN (FRAGMENT)." Database accession no. Q9Y408 XP002202342**
- **DATABASE EMBL [Online] 20 January 1998 (1998-01-20) "Rattus norvegicus tulip 2 mRNA, complete cds." Database accession no. AF041107 XP002202343 -& DATABASE SWISSPROT [Online] 1 June 1998 (1998-06-01) "TULIP 2." Database accession no. O55008 XP002202344**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **DATABASE EMBL [Online] 15 October 1999 (1999-10-15) "Human DNA sequence from clone RP11-287B20 on chromosome 20 Contains part of gene KIAA1272 for a protein similar to rat Tulip 2, ESTs, STSs and GSSs." Database accession no. AL121896 XP002202345 -& DATABASE SWISSPROT [Online] 1 June 2001 (2001-06-01) "BA287B20.1.1 (KIAA1272 SIMILAR TO RAT TULIP PROTEINS 1 AND 2, ISOFORM 1) (FRAGMENT)." Database accession no. Q9BQT6 XP002202346 -& DATABASE SWISSPROT [Online] 1 June 2001 (2001-06-01) "BA287B20.1.2 (KIAA1272 SIMILAR TO RAT TULIP PROTEINS 1 AND 2, ISOFORM 2) (FRAGMENT)." Database accession no. Q9BQT7 XP002202347**
- **DATABASE EMBL [Online] 12 March 1999 (1999-03-12) "Homo sapiens mRNA; EST DKFZp434P1114_r1 (from clone DKFZp434P1114)" Database accession no. AL040683 XP002202348**
- **DATABASE EMBL [Online] 11 November 1999 (1999-11-11) "Homo sapiens mRNA for KIAA1272 protein, partial cds." Database accession no. AB033098 XP002202349 -& DATABASE SWISPROT [Online] 1 May 2000 (2000-05-01) "KIAA1272 PROTEIN (FRAGMENT)." Database accession no. Q9ULE8 XP002202350**
- **CRINO PETER B ET AL: "New developments in the neurobiology of the tuberous sclerosis complex." NEUROLOGY, vol. 53, no. 7, 22 October 1999 (1999-10-22), pages 1384-1390, XP008004619 ISSN: 0028-3878**
- **"Tuberous scelrosis complex in flies too?" NCBI COFFEE BREAK: ARTICLE REFERENCE CB15.270700, [Online] 27 July 2000 (2000-07-27), XP002202338 Retrieved from the Internet: < URL:http://www.ncbi.nlm.nih.gov/Coffeebreak/CB15_gigas/TuberousSclerosis.pdf> [retrieved on 2002-06-14]**

## Description

### Field of the Invention

This invention relates to newly identified polypeptides and polynucleotides encoding such polypeptides sometimes hereinafter referred to as "novel human GTPase (MFQ-111)", to their use in diagnosis and in identifying compounds that may be agonists, antagonists that are potentially useful in therapy, and to production of such polypeptides and polynucleotides.

### Background of the Invention

The drug discovery process is currently undergoing a fundamental revolution as it embraces "functional genomics", that is, high throughput genome- or gene-based biology. This approach as a means to identify genes and gene products as therapeutic targets is rapidly superceding earlier approaches based on "positional cloning". A phenotype, that is a biological function or genetic disease, would be identified and this would then be tracked back to the responsible gene, based on its genetic map position.

Functional genomics relies heavily on high-throughput DNA sequencing technologies and the various tools of bioinformatics to identify gene sequences of potential interest from the many molecular biology databases now available. There is a continuing need to identify and characterize further genes and their related polypeptides/proteins, as targets for drug discovery.

It is well established that many medically significant biological processes are mediated by proteins participating in signal transduction pathways that involve G-proteins and/or second messengers, e.g., cAMP (Lefkowitz, Nature, 1991, 351:353-354). Herein these proteins are referred to as proteins participating in pathways with G-proteins or PPG proteins. Some examples of these proteins include the GPC receptors, such as those for adrenergic agents and dopamine (Kobilka, B.K., et al., Proc. Natl Acad. Sci., USA, 1987, 84:46-50; Kobilka, B.K., et al., Science, 1987, 238:650-656; Bunzow, J.R., et al., Nature, 1988, 336:783-787), G-proteins themselves, effector proteins, e.g., phospholipase C, adenyl cyclase, and phosphodiesterase, and actuator proteins, e.g., protein kinase A and protein kinase C (Simon, M.I., et al., Science, 1991, 252:802-8).

For example, in one form of signal transduction, the effect of hormone binding is the activation of the enzyme, adenylate cyclase, inside the cell. Enzyme activation by hormones is dependent on the presence of the nucleotide GTP. GTP also influences hormone binding. A G-protein connects the hormone receptor to adenylate cyclase. G-protein was shown to exchange GTP for bound GDP when activated by a hormone receptor. The GTP-carrying form then binds to activated adenylate cyclase. Hydrolysis of GTP to GDP, catalyzed by the G-protein itself, returns the G-protein to its basal, inactive form.

Thus the rate of GTP hydrolysis determines the duration between biological activation of the signal pathway and deactivation. The GTPase superfamily comprises G protein signal transducers most notably the products of ras genes that, regulates the rate of cell division (Linder, M. E., et al., 1992, Scientific American, 56:56). Many important cell-surface receptors for hormones and sensory stimuli, for example, transduce extracelular stimuli into cellular responses by promoting formation of the GTP bound state of their GTPase targets. Each protein in the family is a precisely engineered molecular switch that can change its affinities for other macromolecules. Turned on by binding GTP and off by hydrolysing GTP to GDP, the switch mechanism is remarkably versatile, enabling different GTPases to sort and amplify transmembrane signals, direct the synthesis and translocation of proteins, guide vesicular traffic through the cytoplasm, and control proliferation and differentiation of cells. As targets of mutation and microbial toxins, GTPases have pivotal roles in the pathogenesis of cancer and infectious diseases(Boume, H. R., et al., 1991, Nature, 349:117).

Ras is a small guanine nucleotide binding GTPase that transduces biological information from the cell surface to the nucleus. Its ability to transfer growth signals from receptor tyrosine kinases to a mitogen activated protein kinase (MAPK) cascade puts it in the heart of signaling pathways that cause proliferation in normal cells and uncontrolled growth in cancer cells. Indeed, mutations that lock Ras in its active, GTP-bound state lead to malignant transformation and are among the most frequently identified mutations in human cancers (Barbacid, M., Annu. Rev. Biochem., 1987, 56:779; McCormick, F., Nature, 1993, 363:15-16). The Ras family of low molecular weight GTP-binding proteins has been implicated in a wide range of cellular processes, including cell growth and differentiation, intracellular vesicular trafficking, nucleocytoplasmic transport, and cytoskeletonal reorganization (Bourne, H. R., et al., 1990, Nature, 348:125; Zerial, M., et al., Guidebook to the Small GTPases, Oxford University Press, New York (1995).

Another feature of the invention relates to the membrane protein gene superfamily of G-protein coupled receptors characterized as having seven putative transmembrane domains. The domains are believed to represent transmembrane $\alpha$-helices connected by extracellular or cytoplasmic loops. G-protein coupled receptors include a wide range of biologically active receptors, such as hormones, viral, growth factors and neuroreceptors.

G-protein coupled receptors (otherwise known as 7TM receptors) have been characterized as including these seven conserved hydrophobic stretches of about 20 to 30 amino acids, connecting at least eight divergent hydrophilic loops. The G-protein family of coupled receptors includes dopamine receptors which bind to neuroleptic drugs used for treating psychotic and neurological disorders. Other examples of members of this family include, but are not limited to, calcitonin, adrenergic, endothelin, cAMP, adenosine, muscarinic, acetylcholine, serotonin, histamine, thrombin, kinin, follicle stimulating hormone, opsins, endothelial differentiation gene-1, rhodopsins, odorant, and cytomegalovirus receptors.

Most G-protein coupled receptors have single conserved cysteine residues in each of the first two extracellular loops which form disulfide bonds that are believed to stabilize functional protein structure. The 7 transmembrane regions are designated as TM1, TM2, TM3, TM4, TM5, TM6, and TM7. TM3 has been implicated in signal transduction.

G-protein coupled receptors can be intracellularly coupled by heterotrimeric G-proteins to various intracellular enzymes, ion channels and transporters (see, Johnson et al., Endoc. Rev., 1989, 10:317-331) Different G-protein $\alpha$-subunits preferentially stimulate particular effectors to modulate various biological functions in a cell. Phosphorylation of cytoplasmic residues of G-protein coupled receptors have been identified as an important mechanism for the regulation of G-protein coupling of some G-protein coupled receptors. G-protein coupled receptors are found in numerous sites within a mammalian host.

### Summary of the Invention

The present invention relates to MFQ-111, in particular MFQ-111 polypeptides and polynucleotides, recombinant materials and methods for their production. Such polypeptides and polynucleotides are of interest in relation to methods of treatment of certain diseases, including, but not limited to cerebral stroke, heart hypertrophy, heart failure, ischemia, arrhythmia, angina pectoris, hypertension, hypotension, artherosclerosis, restenosis, chronic and acute inflammation, rheumatoid, arthritis, multiple sclerosis, bowel disease, diabetis, cancer, infections such as bacterial, fungal, protozoan and viral infections, particularly infections caused by HIV-1 or HIV-2, pain, obesity, anorexia,bulimia, asthma, Parkinson's disease, urinary retention, osteoporosis, myocardial infarction, ulcers, allergies, benign prostatic hypertrophy, migraine, vomiting, psychotic and neurological disorders including anxiety, schizophrenia, manic depression, depression, delirium, dementia, and severe mental retardation, and dyskinesias, such as Huntington's disease or Gilles dela Tourett's syndrome, hereinafter referred to as " diseases of the invention". In a further aspect, the invention relates to methods for identifying agonists and antagonists (e.g., inhibitors) using the materials provided by the invention, and treating conditions associated with MFQ-111 imbalance with the identified compounds. In a still further aspect, the invention relates to diagnostic assays for detecting diseases associated with inappropriate MFQ-111 activity or levels.

### Description of the Invention

In a first aspect, the present invention relates to MFQ-111 polypeptides. Such polypeptides include:

(a) an polypeptide encoded by a polynucleotide comprising the sequence of SEQ ID NO:1;

(b) a polypeptide comprising a polypeptide sequence having at least 95%, 96%, 97%, 98%, or 99% identity to the polypeptide sequence of SEQ ID NO:2;

(c) a polypeptide comprising the polypeptide sequence of SEQ ID NO:2;

(d) a polypeptide having at least 95%, 96%, 97%, 98%, or 99% identity to the polypeptide sequence of SEQ ID NO:2;

(e) the polypeptide sequence of SEQ ID NO:2; and

(f) a polypeptide having or comprising a polypeptide sequence that has an Identity Index of 0.95, 0.96, 0.97, 0.98, or 0.99 compared to the polypeptide sequence of SEQ ID NO:2;

(g) fragments and variants of such polypeptides in (a) to (f).

Polypeptides of the present invention are believed to be members of the MFQ-111 family of polypeptides. They are therefore of interest because using the subtractive hybridization technique it is possibleto identify differentially expressed genes from a rat stroke model (MCAO). This technology permits to compare two populations of mRNA (or cDNA) and obtain clones of genes that are expressed or overexpressed in one population from another. We use this technology with cDNA derived from mRNA extracted after 2 hours of stroke that contains differentially expressed cDNA (tester). cDNA extracted from sham animals was used as reference cDNA (driver).

The Positive clones were confirmed using a reverse northern analysis. The positive clones derived from the subtracted library were dot-blotted onto nylon membranes and hybridized with second strand cDNA derived from mRNA extracted from brain of sham rats and operated animals 2 and 8 hours after stroke. The clone A3/G12 was identified as differential expressed gene. The insert size of these clone was 333 bp. Analyzing the sequence using BLAST X (J Mol Biol, 1990, 215, 403-410) algorithm the results showed high homology with rat tulip 2 gene at protein level. The human homologous protein was deduced doing manual alignments.

Rat tulip 1 mRNA (AF041106) and rat tulip 2 mRNA (AF041107) have been described previously by Hirao, K and Takai, Y as a tuberin-like protein identified through the yeast two-hybrid screening using rat lin-10 as a bait. The authors have as well described some novel neuronal related proteins (JBC, 1998, 273:3470-3475; JBC, 1998, 273: 21105-21110; JBC, 1998, 273:26269-26272; JBC, 1999, 274:11889-11896; JBC, 1999, 274:27463-27466). Nevertheless a human tulip like protein has not been described yet. More recently Tateno, M and Hayashizaki,Y describes the mouse tulip 1 (AB032400) as a novel member of SNAG repressor family.

The new MFQ-111 gene reveals, using a protein domains database such as Pfam (Nucleic Acids Res., 1999, 27:260-262), a clear Rap-GAP domain and seven putative transmembrane domains. The new human protein thus comprises unexpected elements of the 7-TM protein super family and the small GTPases family.

Tuberin is recently described as a small GTPase protein of the Ras superfamily with a domain Rap-GAP that has an important role in central nervous system disease (Ultrastruc. Pathol., 2000, 24:93-98). The proteins that have the Ras effector domain of small GTPases plays a role in signaling and are regulators of cell proliferation (Curr Opp Cell Biol, 2000, 12:157-165).

RATIONALE

The biological properties of the MFQ-111 are hereinafter referred to as "biological activity of MFQ-111" or "MFQ-111 activity". Preferably, a polypeptide of the present invention exhibits at least one biological activity of MFQ-111.

Polypeptides of the present invention also includes variants of the aforementioned polypeptides, including all allelic forms and splice variants. Such polypeptides vary from the reference polypeptide by insertions, deletions, and substitutions that may be conservative or non-conservative, or any combination thereof. Particularly preferred variants are those in which several, for instance from 50 to 30, from 30 to 20, from 20 to 10, from 10 to 5, from 5 to 3, from 3 to 2, from 2 to 1 or 1 amino acids are inserted, substituted, or deleted, in any combination.

Preferred fragments of polypeptides of the present invention include a polypeptide comprising an amino acid sequence having at least 30, 50 or 100 contiguous amino acids from the amino acid sequence of SEQ ID NO: 2, or a polypeptide comprising an amino acid sequence having at least 30, 50 or 100 contiguous amino acids truncated or deleted from the amino acid sequence of SEQ ID NO: 2. Preferred fragments are biologically active fragments that mediate the biological activity of MFQ-111, including those with a similar activity or an improved activity, or with a decreased undesirable activity. Also preferred are those fragments that are antigenic or immunogenic in an animal, especially in a human.

Fragments of the polypeptides of the invention may be employed for producing the corresponding full-length polypeptide by peptide synthesis; therefore, these variants may be employed as intermediates for producing the full-length polypeptides of the invention.The polypeptides of the present invention may be in the form of the "mature" protein or may be a part of a larger protein such as a precursor or a fusion protein. It is often advantageous to include an additional amino acid sequence that contains secretory or leader sequences, pro-sequences, sequences that aid in purification, for instance multiple histidine residues, or an additional sequence for stability during recombinant production.

Polypeptides of the present invention can be prepared in any suitable manner, for instance by isolation form naturally occurring sources, from genetically engineered host cells comprising expression systems (*vide infra)* or by chemical synthesis, using for instance automated peptide synthesizers, or a combination of such methods. Means for preparing such polypeptides are well understood in the art.

In a further aspect, the present invention relates to MFQ-111 polynucleotides. Such polynucleotides include:

(a) a polynucleotide comprising a polynucleotide sequence having at least 95%, 96%, 97%, 98%, or 99% identity to the polynucleotide squence of SEQ ID NO:1;

(b) a polynucleotide comprising the polynucleotide of SEQ ID NO:1;

(c) a polynucleotide having at least 95%, 96%, 97%, 98%, or 99% identity to the polynucleotide of SEQ ID NO:1;

(d) the polynucleotide of SEQ ID NO:1;

(e) a polynucleotide comprising a polynucleotide sequence encoding a polypeptide sequence having at least 95%, 96%, 97%, 98%, or 99% identity to the polypeptide sequence of SEQ ID NO:2;

(f) a polynucleotide comprising a polynucleotide sequence encoding the polypeptide of SEQ ID NO:2;

(g) a polynucleotide having a polynucleotide sequence encoding a polypeptide sequence having at least 95%, 96%, 97%, 98%, or 99% identity to the polypeptide sequence of SEQ ID NO:2;

(h) a polynucleotide encoding the polypeptide of SEQ ID NO:2;

(i) a polynucleotide having or comprising a polynucleotide sequence that has an Identity Index of 0.95, 0.96, 0.97, 0.98, or 0.99 compared to the polynucleotide sequence of SEQ ID NO:1;

(j) a polynucleotide having or comprising a polynucleotide sequence encoding a polypeptide sequence that has an Identity Index of 0.95, 0.96, 0.97, 0.98, or 0.99 compared to the polypeptide sequence of SEQ ID NO:2; and polynucleotides that are fragments and variants of the above mentioned polynucleotides or that are complementary to above mentioned polynucleotides, over the entire length thereof.

Preferred fragments of polynucleotides of the present invention include a polynucleotide comprising an nucleotide sequence having at least 15, 30, 50 or 100 contiguous nucleotides from the sequence of SEQ ID NO: 1, or a polynucleotide comprising an sequence having at least 30, 50 or 100 contiguous nucleotides truncated or deleted from the sequence of SEQ ID NO: 1.

Preferred variants of polynucleotides of the present invention include splice variants, allelic variants, and polymorphisms, including polynucleotides having one or more single nucleotide polymorphisms (SNPs).

Polynucleotides of the present invention also include polynucleotides encoding polypeptide variants that comprise the amino acid sequence of SEQ ID NO:2 and in which several, for instance from 50 to 30, from 30 to 20, from 20 to 10, from 10 to 5, from 5 to 3, from 3 to 2, from 2 to 1 or 1 amino acid residues are substituted, deleted or added, in any combination.

In a further aspect, the present invention provides polynucleotides that are RNA transcripts of the DNA sequences of the present invention. Accordingly, there is provided an RNA polynucleotide that:

(a) comprises an RNA transcript of the DNA sequence encoding the polypeptide of SEQ ID NO:2;

(b) is the RNA transcript of the DNA sequence encoding the polypeptide of SEQ ID NO:2;

(c) comprises an RNA transcript of the DNA sequence of SEQ ID NO:1; or

(d) is the RNA transcript of the DNA sequence of SEQ ID NO:1;

and RNA polynucleotides that are complementary thereto.

The polynucleotide sequence of SEQ ID NO:1 shows similarity with R.norvegicus tulip 1 (AF041106), R.norvegicus tulip 2(AF041107), M.musculus tulip1 (AB032400), H.sapiens cDNA DKFZp566D133 (HSM800380).

The polynucleotide sequence of SEQ ID NO:1 also shows similarity with the following sequences listed in the EMBL Database: H. sapiens cDNA AL050050, and H. sapiens DNA AL158070, from clone RP11-235C23 on chromosome 9, H. sapiens cDNA AL040683. WO9518226 discloses the use of the TSC2 gene for the diagnosis of cancer.

The human DNA sequence AL158070 contains a pseudogene, which shows 99% identity to the current SEQ ID NO:1, located at chromosome 9, while the gene of the inventive protein is located on chromosome 14.

The rat sequence of AF041107 describes a 824 amino acid protein a rat protein which is different from the 861 amino acids protein of the current application. The rat gene is located on chromosome 9.

H. sapiens cDNA AL050050 describes a human hypothetical 43.9KDA protein fragment with a total of 381 amino acids, which may be derived from kidney however no further information is linked to this sequence.

. The polynucleotide sequence of SEQ ID NO:1 is a cDNA sequence that encodes the polypeptide of SEQ ID NO:2. The polynucleotide sequence encoding the polypeptide of SEQ ID NO:2 may be identical to the polypeptide encoding sequence of SEQ ID NO:1 or it may be a sequence other than SEQ ID NO:1, which, as a result of the redundancy (degeneracy) of the genetic code, also encodes the polypeptide of SEQ ID NO:2. The polypeptide of the SEQ ID NO:2 is related to other proteins of the MFQ-111 family, having homology and/or structural similarity with R.norvegicus Tulip 2(AAB97076), R.norvegicus Tulip1 (AAB97075), H.sapiens KIAA1272 (BAA8586).

Preferred polypeptides and polynucleotides of the present invention are expected to have, *inter alia,* similar biological functions/properties to their homologous polypeptides and polynucleotides. Furthermore, preferred polypeptides and polynucleotides of the present invention have at least one MFQ-111 activity.

Polynucleotides of the present invention may be obtained using standard cloning and screening techniques from a cDNA library derived from mRNA in cells of human brain, (see for instance, Sambrook *et al.*, Molecular Cloning: A Laboratory Manual, 2nd Ed., Cold Spring Harbor Laboratory Press, Cold Spring Harbor, N.Y. (1989). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself, or the coding sequence for the mature polypeptide in reading frame with other coding sequences, such as those encoding a leader or secretory sequence, a pre-, or pro- or prepro- protein sequence, or other fusion peptide portions. For example, a marker sequence that facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al.*, Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Polynucleotides that are identical, or have sufficient identity to a polynucleotide sequence of SEQ ID NO:1, may be used as hybridization probes for cDNA and genomic DNA or as primers for a nucleic acid amplification reaction (for instance, PCR). Such probes and primers may be used to isolate full-length cDNAs and genomic clones encoding polypeptides of the present invention and to isolate cDNA and genomic clones of other genes (including genes encoding paralogs from human sources and orthologs and paralogs from species other than MFQ-111) that have a high sequence similarity to SEQ ID NO:1, typically at least 95% identity. Preferred probes and primers will generally comprise at least 15 nucleotides, preferably, at least 30 nucleotides and may have at least 50, if not at least 100 nucleotides. Particularly preferred probes will have between 30 and 50 nucleotides. Particularly preferred primers will have between 20 and 25 nucleotides.

A polynucleotide encoding a polypeptide of the present invention, including homologs from species other than MFQ-111, may be obtained by a process comprising the steps of screening a library under stringent hybridization conditions with a labeled probe having the sequence of SEQ ID NO: 1 or a fragment thereof, preferably of at least 15 nucleotides; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA; followed by washing the filters in 0.1x SSC at about 65°C. Thus the present invention also includes isolated polynucleotides, preferably with a nucleotide sequence of at least 100, obtained by screening a library under stringent hybridization conditions with a labeled probe having the sequence of SEQ ID NO:1 or a fragment thereof, preferably of at least 15 nucleotides.

The skilled artisan will appreciate that, in many cases, a cDNA sequence will be incomplete, in that the region coding for the polypeptide does not extend all the way through to the 5' terminus. This is a consequence of reverse transcriptase, an enzyme with inherently low "processivity" (a measure of the ability of the enzyme to remain attached to the template during the polymerisation reaction), failing to complete a DNA copy of the mRNA template during first strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., Proc Nat Acad Sci USA 85, 8998-9002, 1988). Recent modifications of the technique, exemplified by the Marathon (trade mark) technology (Clontech Laboratories Inc.) for example, have significantly simplified the search for longer cDNAs. In the Marathon (trade mark) technology, cDNAs have been prepared from mRNA extracted from a chosen tissue and an 'adaptor' sequence ligated onto each end. Nucleic acid amplification (PCR) is then carried out to amplify the "missing" 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, in a further aspect, the present invention relates to expression systems comprising a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Polynucleotides may be introduced into host cells by methods described in many standard laboratory manuals, such as Davis et al., Basic Methods in Molecular Biology (1986) and Sambrook *et al.*(*ibid*). Preferred methods of introducing polynucleotides into host cells include, for instance, calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector that is able to maintain, propagate or express a polynucleotide to produce a polypeptide in a host may be used. The appropriate polynucleotide sequence may be inserted into an expression system by any of a variety of well-known and routine techniques, such as, for example, those set forth in Sambrook *et al.*, (*ibid*). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and/or purification.

Polynucleotides of the present invention may be used as diagnostic reagents, through detecting mutations in the associated gene. Detection of a mutated form of the gene characterised by the polynucleotide of SEQ ID NO:1 in the cDNA or genomic sequence and which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques well known in the art.

Nucleic acids for diagnosis may be obtained from a subject's cells, such as from blood, urine, saliva, tissue biopsy or autopsy material. The genomic DNA may be used directly for detection or it may be amplified enzymatically by using PCR, preferably RT-PCR, or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled MFQ-111 nucleotide sequences. Perfectly matched sequences can be distinguished from mismatched duplexes by RNase digestion or by differences in melting temperatures. DNA sequence difference may also be detected by alterations in the electrophoretic mobility of DNA fragments in gels, with or without denaturing agents, or by direct DNA sequencing (see, for instance, Myers *et al.*, Science (1985) 230:1242). Sequence changes at specific locations may also be revealed by nuclease protection assays, such as RNase and S1 protection or the chemical cleavage method (see Cotton *et al.*, Proc Natl Acad Sci USA (1985) 85: 4397-4401).

An array of oligonucleotides probes comprising MFQ-111 polynucleotide sequence or fragments thereof can be constructed to conduct efficient screening of *e.g.*, genetic mutations. Such arrays are preferably high density arrays or grids. Array technology methods are well known and have general applicability and can be used to address a variety of questions in molecular genetics including gene expression, genetic linkage, and genetic variability, see, for example, M.Chee et al., Science, 274, 610-613 (1996) and other references cited therein.

Detection of abnormally decreased or increased levels of polypeptide or mRNA expression may also be used for diagnosing or determining susceptibility of a subject to a disease of the invention. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention relates to a diagonostic kit comprising:

(a) a polynucleotide of the present invention, preferably the nucleotide sequence of SEQ ID NO: 1, or a fragment or an RNA transcript thereof;

(b) a nucleotide sequence complementary to that of (a);

(c) a polypeptide of the present invention, preferably the polypeptide of SEQ ID NO:2 or a fragment thereof; or

(d) an antibody to a polypeptide of the present invention, preferably to the polypeptide of SEQ ID NO:2.

It will be appreciated that in any such kit, (a), (b), (c) or (d) may comprise a substantial component. Such a kit will be of use in diagnosing a disease or susceptibility to a disease, particularly diseases of the invention, amongst others.

The polynucleotide sequences of the present invention are valuable for chromosome localisation studies. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (co-inheritance of physically adjacent genes). Precise human chromosomal localisations for a genomic sequence (gene fragment etc.) can be determined using Radiation Hybrid (RH) Mapping (Walter, M. Spillett, D., Thomas, P., Weissenbach, J., and Goodfellow, P., (1994) A method for constructing radiation hybrid maps of whole genomes, Nature Genetics 7, 22-28). A number of RH panels are available from Research Genetics (Huntsville, AL, USA) e.g. the GeneBridge4 RH panel (Hum Mol Genet 1996 Mar;5(3):339-46 A radiation hybrid map of the human genome. Gyapay G, Schmitt K, Fizames C, Jones H, Vega-Czarny N, Spillett D, Muselet D, Prud'Homme JF, Dib C, Auffray C, Morissette J, Weissenbach J, Goodfellow PN). To determine the chromosomal location of a gene using this panel, 93 PCRs are performed using primers designed from the gene of interest on RH DNAs. Each of these DNAs contains random human genomic fragments maintained in a hamster background (human / hamster hybrid cell lines). These PCRs result in 93 scores indicating the presence or absence of the PCR product of the gene of interest. These scores are compared with scores created using PCR products from genomic sequences of known location. This comparison is conducted at http://www.genome.wi.mit.edu/. The gene of the present invention maps to human chromosome 14.

The polynucleotide sequences of the present invention are also valuable tools for tissue expression studies. Such studies allow the determination of expression patterns of polynucleotides of the present invention which may give an indication as to the expression patterns of the encoded polypeptides in tissues, by detecting the mRNAs that encode them. The techniques used are well known in the art and include in situ hydridisation techniques to clones arrayed on a grid, such as cDNA microarray hybridisation (Schena *et al*, Science, 270, 467-470, 1995 and Shalon *et al*, Genome Res, 6, 639-645, 1996) and nucleotide amplification techniques such as PCR. A preferred method uses the TAQMAN (Trade mark) technology available from Perkin Elmer. Results from these studies can provide an indication of the normal function of the polypeptide in the organism. In addition, comparative studies of the normal expression pattern of mRNAs with that of mRNAs encoded by an alternative form of the same gene (for example, one having an alteration in polypeptide coding potential or a regulatory mutation) can provide valuable insights into the role of the polypeptides of the present invention, or that of inappropriate expression thereof in disease. Such inappropriate expression may be of a temporal, spatial or simply quantitative nature.

The polypeptides of the present invention are expressed in brain, amygdala, cerebellum, infant brain, heart, placenta, lung, fetal lung, liver, skeletal muscle, kidney, pancreas, thymus, prostate, testis, ovary , T-cell, fetal live spleen, endometrial tumor, testis tumor, breast carcinoma, lung carcinoma, colon adenocarcinoma and pancreatic adenocarcinoma.

Polypeptides and polynucleotides of the present invention may also be used as vaccines. Accordingly, in a further aspect, the present invention relates to a method for inducing an immunological response in a mammal that comprises inoculating the mammal with a polypeptide of the present invention, adequate to produce antibody and/or T cell immune response, including, for example, cytokine-producing T cells or cytotoxic T cells, to protect said animal from disease, whether that disease is already established within the individual or not. An immunological response in a mammal may also be induced by a method comprises delivering a polypeptide of the present invention *via* a vector directing expression of the polynucleotide and coding for the polypeptide *in vivo* in order to induce such an immunological response to produce antibody to protect said animal from diseases of the invention. One way of administering the vector is by accelerating it into the desired cells as a coating on particles or otherwise. Such nucleic acid vector may comprise DNA, RNA, a modified nucleic acid, or a DNA/RNA hybrid. For use a vaccine, a polypeptide or a nucleic acid vector will be normally provided as a vaccine formulation (composition). The formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions that may contain anti-oxidants, buffers, bacteriostats and solutes that render the formulation instonic with the blood of the recipient; and aqueous and non-aqueous sterile suspensions that may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention have one or more biological functions that are of relevance in one or more disease states, in particular the diseases of the invention hereinbefore mentioned. It is therefore useful to to identify compounds that stimulate or inhibit the function or level of the polypeptide. Accordingly, in a further aspect, the present invention provides for a method of screening compounds to identify those that stimulate or inhibit the function or level of the polypeptide. Such methods identify agonists or antagonists that may be employed for therapeutic and prophylactic purposes for such diseases of the invention as hereinbefore mentioned. Compounds may be identified from a variety of sources, for example, cells, cell-free preparations, chemical libraries, collections of chemical compounds, and natural product mixtures. Such agonists or antagonists so-identified may be natural or modified substrates, ligands, receptors, enzymes, etc., as the case may be, of the polypeptide; a structural or functional mimetic thereof (see Coligan *et al.*, Current Protocols in Immunology 1 (2):Chapter 5 (1991)) or a small molecule.

The screening method may simply measure the binding of a candidate compound to the polypeptide, or to cells or membranes bearing the polypeptide, or a fusion protein thereof, by means of a label directly or indirectly associated with the candidate compound.

Another methods for detecting agonists or antagonists for the receptor of the present invention is the yeast based technology as described in U.S. Patent 5,482,835.

One screening technique includes the use of cells which express receptor of this invention (for example, transfected CHO cells) in a system which measures extracellular pH or intracellular calcium changes caused by receptor activation. In this technique, compounds may be contacted with cells expressing the receptor polypeptide of the present invention. A second messenger response, e.g., signal transduction, pH changes, or changes in calcium level, is then measured to determine whether the potential compound activates or inhibits the receptor.

Another method involves screening for receptor inhibitors by determining inhibition or stimulation of receptor-mediated cAMP and/or adenylate cyclase accumulation. Such a method involves transfecting a eukaryotic cell with the receptor of this invention to express the receptor on the cell surface. The cell is then exposed to potential antagonists in the presence of the receptor of this invention. The amount of cAMP accumulation is then measured. If the potential antagonist binds the receptor, and thus inhibits receptor binding, the levels of receptor-mediated cAMP, or adenylate cyclase, activity will be reduced or increased.

Another methods for detecting agonists or antagonists for the receptor of the present invention is the yeast based technology as described in U.S. Patent 5,482,835.

Screening techniques

The polynucleotides, polypeptides and antibodies to the polypeptide of the present invention may also be used to configure screening methods for detecting the effect of added compounds on the production of mRNA and polypeptide in cells. For example, an ELISA assay may be constructed for measuring secreted or cell associated levels of polypeptide using monoclonal and polyclonal antibodies by standard methods known in the art. This can be used to discover agents that may inhibit or enhance the production of polypeptide (also called antagonist or agonist, respectively) from suitably manipulated cells or tissues.

A polypeptide of the present invention may be used to identify membrane bound or soluble receptors, if any, through standard receptor binding techniques known in the art. These include, but are not limited to, ligand binding and crosslinking assays in which the polypeptide is labeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide that compete with the binding of the polypeptide to its receptors, if any. Standard methods for conducting such assays are well understood in the art.

Examples of antagonists of polypeptides of the present invention include antibodies or, in some cases, oligonucleotides or proteins that are closely related to the ligands, substrates, receptors, enzymes, etc., as the case may be, of the polypeptide, *e.g.*, a fragment of the ligands, substrates, receptors, enzymes, etc.; or a small molecule that bind to the polypeptide of the present invention but do not elicit a response, so that the activity of the polypeptide is prevented.

Screening methods may also involve the use of transgenic technology and MFQ-111 gene. The art of constructing transgenic animals is well established. For example, the MFQ-111 gene may be introduced through microinjection into the male pronucleus of fertilized oocytes, retroviral transfer into pre- or post-implantation embryos, or injection of genetically modified, such as by electroporation, embryonic stem cells into host blastocysts. Particularly useful transgenic animals are so-called "knock-in" animals in which an animal gene is replaced by the human equivalent within the genome of that animal. Knock-in transgenic animals are useful in the drug discovery process, for target validation, where the compound is specific for the human target. Other useful transgenic animals are so-called "knock-out" animals in which the expression of the animal ortholog of a polypeptide of the present invention and encoded by an endogenous DNA sequence in a cell is partially or completely annulled. The gene knock-out may be targeted to specific cells or tissues, may occur only in certain cells or tissues as a consequence of the limitations of the technology, or may occur in all, or substantially all, cells in the animal.

Transgenic animal technology also offers a whole animal expression-cloning system in which introduced genes are expressed to give large amounts of polypeptides of the present invention

Screening kits for use in the above described methods form a further aspect of the present invention. Such screening kits comprise:

(a) a polypeptide of the present invention;

(b) a recombinant cell expressing a polypeptide of the present invention;

(c) a cell membrane expressing a polypeptide of the present invention; or

(d) an antibody to a polypeptide of the present invention;

which polypeptide is preferably that of SEQ ID NO:2.

It will be appreciated that in any such kit, (a), (b), (c) or (d) may comprise a substantial component.

**Glossary**

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an

Fab or other immunoglobulin expression library.

"Isolated" means altered "by the hand of man" from its natural state, *i.e.*, if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide" generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any polypeptide comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications.

Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter *et al.*, "Analysis for protein modifications and nonprotein cofactors", Meth Enzymol, 182, 626-646, 1990, and Rattan *et al.*, "Protein Synthesis: Post-translational Modifications and Aging", Ann NY Acad Sci, 663, 48-62, 1992).

"Fragment" of a polypeptide sequence refers to a polypeptide sequence that is shorter than the reference sequence but that retains essentially the same biological function or activity as the reference polypeptide. "Fragment" of a polynucleotide sequence refers to a polynucloetide sequence that is shorter than the reference sequence of SEQ ID NO:1..

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines.

"Allele" refers to one of two or more alternative forms of a gene occuring at a given locus in the genome.

"Polymorphism" refers to a variation in nucleotide sequence (and encoded polypeptide sequence, if relevant) at a given position in the genome within a population.

"Single Nucleotide Polymorphism" (SNP) refers to the occurence of nucleotide variability at a single nucleotide position in the genome, within a population. An SNP may occur within a gene or within intergenic regions of the genome. SNPs can be assayed using Allele Specific Amplification (ASA). For the process at least 3 primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the Allele Specific Primers.

"Splice Variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one mRNA molecule each of that may encode different amino acid sequences. The term splice variant also refers to the proteins encoded by the above cDNA molecules.

"Identity" reflects a relationship between two or more polypeptide sequences or two or more polynucleotide sequences, determined by comparing the sequences. In general, identity refers to an exact nucleotide to nucleotide or amino acid to amino acid correspondence of the two polynucleotide or two polypeptide sequences, respectively, over the length of the sequences being compared.

"% Identity" - For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

"Similarity" is a further, more sophisticated measure of the relationship between two polypeptide sequences. In general, "similarity" means a comparison between the amino acids of two polypeptide chains, on a residue by residue basis, taking into account not only exact correspondences between a between pairs of residues, one from each of the sequences being compared (as for identity) but also, where there is not an exact correspondence, whether, on an evolutionary basis, one residue is a likely substitute for the other. This likelihood has an associated "score" from which the "% similarity" of the two sequences can then be determined.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (J Mol Biol, 147,195-197, 1981, Advances in Applied Mathematics, 2, 482-489, 1981) and finds the best single region of similarity between two sequences. BESTFIT is more suited to comparing two polynucleotide or two polypeptide sequences that are dissimilar in length, the program assuming that the shorter sequence represents a portion of the longer. In comparison, GAP aligns two sequences, finding a "maximum similarity", according to the algorithm of Needleman and Wunsch (J Mol Biol, 48, 443-453, 1970). GAP is more suited to comparing sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R, Methods in Enzymology, 183, 63-99, 1990; Pearson W R and Lipman D J, Proc Nat Acad Sci USA, 85, 2444-2448,1988, available as part of the Wisconsin Sequence Analysis Package).

Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a reference polynucleotide or a polypeptide sequence, the query and the reference sequence being optimally aligned and the parameters of the program set at the default value, as hereinbefore described.

"Identity Index" is a measure of sequence relatedness which may be used to compare a candidate sequence (polynucleotide or polypeptide) and a reference sequence. Thus, for instance, a candidate polynucleotide sequence having, for example, an Identity Index of 0.95 compared to a reference polynucleotide sequence is identical to the reference sequence except that the candidate polynucleotide sequence may include on average up to five differences per each 100 nucleotides of the reference sequence. Such differences are selected from the group consisting of at least one nucleotide deletion, substitution, including transition and transversion, or insertion. These differences may occur at the 5' or 3' terminal positions of the reference polynucleotide sequence or anywhere between these terminal positions, interspersed either individually among the nucleotides in the reference sequence or in one or more contiguous groups within the reference sequence. In other words, to obtain a polynucleotide sequence having an Identity Index of 0.95 compared to a reference polynucleotide sequence, an average of up to 5 in every 100 of the nucleotides of the in the reference sequence may be deleted, substituted or inserted, or any combination thereof, as hereinbefore described. The same applies *mutatis mutandis* for other values of the Identity Index, for instance 0.96, 0.97, 0.98 and 0.99.

Similarly, for a polypeptide, a candidate polypeptide sequence having, for example, an Identity Index of 0.95 compared to a reference polypeptide sequence is identical to the reference sequence except that the polypeptide sequence may include an average of up to five differences per each 100 amino acids of the reference sequence. Such differences are selected from the group consisting of at least one amino acid deletion, substitution, including conservative and non-conservative substitution, or insertion. These differences may occur at the amino- or carboxy-terminal positions of the reference polypeptide sequence or anywhere between these terminal positions, interspersed either individually among the amino acids in the reference sequence or in one or more contiguous groups within the reference sequence. In other words, to obtain a polypeptide sequence having an Identity Index of 0.95 compared to a reference polypeptide sequence, an average of up to 5 in every 100 of the amino acids in the reference sequence may be deleted, substituted or inserted, or any combination thereof, as hereinbefore described. The same applies *mutatis mutandis* for other values of the Identity Index, for instance 0.96, 0.97, 0.98 and 0.99.

The relationship between the number of nucleotide or amino acid differences and the Identity Index may be expressed in the following equation:

$$n_a \leq x_a - (x_a \bullet I),$$

in which:

$\mathbf{n_a}$ is the number of nucleotide or amino acid differences,

$\mathbf{x_a}$ is the total number of nucleotides or amino acids in SEQ ID NO:1 or SEQ ID NO:2, respectively,

**I** is the Identity Index ,

• is the symbol for the multiplication operator, and

in which any non-integer product of $\mathbf{x_a}$ and **I** is rounded down to the nearest integer prior to subtracting it from $\mathbf{x_a}$.

"Homolog" is a generic term used in the art to indicate a polynucleotide or polypeptide sequence possessing a high degree of sequence relatedness to a reference sequence. Such relatedness may be quantified by determining the degree of identity and/or similarity between the two sequences as hereinbefore defined. Falling within this generic term are the terms "ortholog", and "paralog". "Ortholog" refers to a polynucleotide or polypeptide that is the functional equivalent of the polynucleotide or polypeptide in another species. "Paralog" refers to a polynucleotideor polypeptide that within the same species which is functionally similar:

**Further Examples**

Example 1: Mammalian Cell Expression

The receptors of the present invention are expressed in either human embryonic kidney 293 (HEK293) cells or adherent dhfr CHO cells. To maximize receptor expression, typically all 5' and 3' untranslated regions (UTRs) are removed from the receptor cDNA prior to insertion into a pCDN or pCDNA3 vector. The cells are transfected with individual receptor cDNAs by lipofectin and selected in the presence of 400 mg/ml G418. After 3 weeks of selection, individual clones are picked and expanded for further analysis. HEK293 or CHO cells transfected with the vector alone serve as negative controls. To isolate cell lines stably expressing the individual receptors, about 24 clones are typically selected and analyzed by Northern blot analysis. Receptor mRNAs are generally detectable in about 50% of the G418-resistant clones analyzed.

Example 2 Ligand bank for binding and functional assays.

A bank of over 600 putative receptor ligands has been assembled for screening. The bank comprises: transmitters, hormones and chemokines known to act via a human seven transmembrane (7TM) receptor; naturally occurring compounds which may be putative agonists for a human 7TM receptor, non-mammalian, biologically active peptides for which a mammalian counterpart has not yet been identified; and compounds not found in nature, but which activate 7TM receptors with unknown natural ligands. This bank is used to initially screen the receptor for known ligands, using both functional (i.e . calcium, cAMP, microphysiometer, oocyte electrophysiology, etc, see below) as well as binding assays.

Example 3: Ligand Binding Assays

Ligand binding assays provide a direct method for ascertaining receptor pharmacology and are adaptable to a high throughput format. The purified ligand for a receptor is radiolabeled to high specific activity (50-2000 Ci/mmol) for binding studies. A determination is then made that the process of radiolabeling does not diminish the activity of the ligand towards its receptor. Assay conditions for buffers, ions, pH and other modulators such as nucleotides are optimized to establish a workable signal to noise ratio for both membrane and whole cell receptor sources. For these assays, specific receptor binding is defined as total associated radioactivity minus the radioactivity measured in the presence of an excess of unlabeled competing ligand. Where possible, more than one competing ligand is used to define residual nonspecific binding.

Example 4: Functional Assay in Xenopus Oocytes

Capped RNA transcripts from linearized plasmid templates encoding the receptor cDNAs of the invention are synthesized in vitro with RNA polymerases in accordance with standard procedures. In vitro transcripts are suspended in water at a final concentration of 0.2 mg/ml. Ovarian lobes are removed from adult female toads, Stage V defolliculated oocytes are obtained, and RNA transcripts (10 ng/oocyte) are injected in a 50 nl bolus using a microinjection apparatus. Two electrode voltage clamps are used to measure the currents from individual Xenopus oocytes in response to agonist exposure. Recordings are made in Ca2+ free Barth's medium at room temperature. The Xenopus system can be used to screen known ligands and tissue/cell extracts for activating ligands.

Example 5: Microphysiometric Assays

Activation of a wide variety of secondary messenger systems results in extrusion of small amounts of acid from a cell. The acid formed is largely as a result of the increased metabolic activity required to fuel the intracellular signaling process. The pH changes in the media surrounding the cell are very small but are detectable by the CYTOSENSOR microphysiometer (Molecular Devices Ltd., Menlo Park, CA). The CYTOSENSOR is thus capable of detecting the activation of a receptor which is coupled to an energy utilizing intracellular signaling pathway such as the G-protein coupled receptor of the present invention.

Example 6: Extract/Cell Supernatant Screening

A large number of mammalian receptors exist for which there remains, as yet, no cognate activating ligand (agonist). Thus, active ligands for these receptors may not be included within the ligands banks as identified to date. Accordingly, the 7TM receptor of the invention is also functionally screened (using calcium, cAMP, microphysiometer, oocyte electrophysiology, etc., functional screens) against tissue extracts to identify natural ligands. Extracts that produce positive functional responses can be sequencially subfractionated until an activating ligand is isolated identified.

Example 7: Calcium and cAMP Functional Assays

7TM receptors which are expressed in HEK 293 cells have been shown to be coupled functionally to activation of PLC and calcium mobilization and/or cAMP stimuation or inhibition. Basal calcium levels in the HEK 293 cells in receptor-transfected or vector control cells were observed to be in the normal, 100 nM to 200 nM, range. HEK 293 cells expressing recombinant receptors are loaded with fura 2 and in a single day > 150 selected ligands or tissue/cell extracts are evaluated for agonist induced calcium mobilization. Similarly, HEK 293 cells expressing recombinant receptors are evaluated for the stimulation or inhibition of cAMP production using standard cAMP quantitation assays. Agonists presenting a calcium transient or cAMP flucuation are tested in vector control cells to determine if the response is unique to the transfected cells expressing receptor.

Exemple 8: PCR amplification fo MFQ-111

PCR amplification of human tulip homologue: In order to confirm the MFQ-111 sequence a pair of specific PCR primers (see SEQ ID No. 3 and 4) were designed based on the putative sequence:

MFQ-111 upper: 5'.- 3' Primer01 (SEQ ID No. 3)

MFQ-111 lower: 5' - 3' Primer02 (SEQ ID No. 4)

3'

The expected 523 bp fragment was amplified from a human brain cDNA sample (Clontech Ref: 7187-1; whole brain sample of Human MTC panel I from Clontech , Ref: K1420-1) and the sequence was confirmed after cloning the correct size PCR band in a pCRII vector from Invitrogen.

**Figure 1**

PCR amplification of specific MFQ-111 band

Lanes:

1- G3PDH control

2- Tulip homologue amplification from human brain cDNA(7187-1)

3- Tulip homologue amplification from brain cDNA (from MTC panel)

4- Negative control

5- Negative control

6- 100 bp ladder

The PCR conditions were : 9 min at 95 °C; 30 sec at 95°C, 50 sec at 54 °C, 60 sec at 72°C for 34 cycles and a final elongation step at 72°C for 5 min using the Taq Gold polymerase purchased from Perkin Elmer.

**Figure 2**

Tissue distribution

With the MFQ-111 designed primers mentioned aboved, and the same PCR conditions a 523 bp specific PCR band is amplified using a set of human cDNA (Human MTC panel I from Clontech, Ref K1420-1 and Human MTC panel II from Clontech, Ref K1421-1).

Specific primers for the house keeping gene G3PDH are used (G3PDH upstream: 5'-3' Primer03 (SEQ ID No. 5) and G3PDH downstream 5' - 3' Primer04 (SEQ ID No. 6) and a control.

Lanes:

1- 100 bp ladder

2- heart

3- brain (whole)

4- placenta

5- lung

6- liver

7- skeletal musclse

8- kindey

9- pancreas

10- spleen

11- thymus

12- prostate

13- testis

14- ovary

15- small intestine

16- colon

17- peripheral blood leukocyte

18- 1 kb ladder

19- positive control amplification MFQ-111 from human brain cDNA

20- positive control amplification of G3PDH

MFQ-111 is not expressed in small intestine and normal colon. The expression level is very week in spleen, and in peripheral blood leukocyte.

**Figure 3**

Expression in Human Tumors

The same PCR strategy is used to determine the level of expression of the gene of interest in several normalized human tumoral tissues using the human tumor multiple tissue cDNA panel from Clontech (Ref K1422-1)

Lanes:

1 100 pb ladder

2- human breast carcinoma

3- human lung carcinoma

4- human colon adenocarcinoma

5- human lung carcinoma

6- human prostatic adenocarcinoma

7- human colon adenocarcinoma

8- human ovarian carcinoma

9- human pancreatic adenocarninoma

10- 1 Kb ladder

11- positive control amplification MFQ-111 from human brain cDNA

12- positive control amplification of G3PDH

13- nevative control primers MFQ-111

14- negative control primers G3PDH

15- 100 pb ladder

MFQ-111 have not a high expression in tumors. Is not expressed in several lung carcinoma and in prostatic adenocarcinoma.

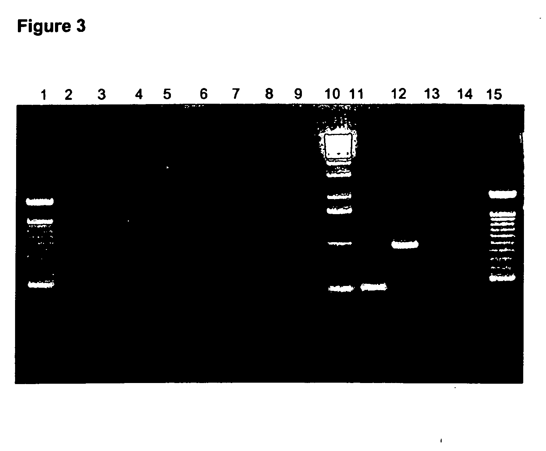

**Claims**

1. An isolated polypeptide selected from one of the groups consisting of:

   (a) an isolated polypeptide encoded by a polynucleotide having the sequence of SEQ ID NO:1;
   (b) an isolated polypeptide comprising a polypeptide sequence having at least 95% identity to the entire polypeptide sequence of SEQ ID NO:2;
   c) an isolated polypeptide having at least 95% identity to the entire polypeptide sequence of SEQ ID NO:2; and the polypeptide sequence of SEQ ID NO:2

2. The isolated polypeptide as claimed in claim 1 comprising the polypeptide sequence of SEQ ID NO:2.

3. The isolated polypeptide as claimed in claim 1 which is the polypeptide sequence of SEQ ID NO:2.

4. An isolated polynucleotide selected from one of the groups consisting of:

   (a) an isolated polynucleotide consisting of a sequence having at least 95% identity, over the entire length, to the polynucleotide of SEQ ID NO:1;

(b) an isolated polynucleotide having a polynucleotide sequence encoding a polypeptide sequence having at least 95% identity, over the entire length, to the polypeptide sequence of SEQ ID NO:2;
(c) an isolated polynucleotide which is the RNA equivalent of a polynucleotide of (a) to (b);

or a polynucleotide sequence complementary to said isolated polynucleotide.

**5.** A recombinant host cell comprising the expression vector encoding the polypeptide of any one of claims 1-3.

**Patentansprüche**

**1.** Isoliertes Polypeptid, ausgewählt aus einer der Gruppen bestehend aus:

(a) einem isolierten Polypeptid, das von einem Polynucleotid mit der Sequenz der SEQ ID NR: 1 kodiert wird;
(b) einem isolierten Polypeptid, das eine Polypeptidsequenz mit mindestens 95% Identität zu der gesamten Polypeptidsequenz der SEQ ID NR: 2 umfasst;
(c) einem isolierten Polypeptid, das mindestens 95% Identität mit der gesamten Polypeptidsequenz der SEQ ID NR: 2 besitzt; und der Polypeptidsequenz der SEQ ID NR: 2.

**2.** Isoliertes Polypeptid nach Anspruch 1, das die Polypeptidsequenz der SEQ ID NR: 2 enthält.

**3.** Isoliertes Polypeptid nach Anspruch 1, bei dem es sich um das Polypeptid der SEQ ID NR: 2 handelt.

**4.** Isoliertes Polypeptid, ausgewählt aus einer der Gruppen bestehend aus:

(a) einem isolierten Polynucleotid, das aus einer Sequenz mit mindestens 95% Identität über die gesamte Länge zu dem Polynucleotid der SEQ ID NR: 1 besteht;
(b) einem isolierten Polynucleotid mit einer Polynucleotidsequenz, die für eine Polypeptidsequenz mit mindestens 95% Identität über die gesamte Länge zu der Polypeptidsequenz der SEQ ID NR: 2 kodiert;
(c) einem isolierten Polynucleotid, bei dem es sich um das RNA-Äquivalent eines Polynucleotids von (a) bis (b) handelt;

oder einer zu diesem isolierten Polynucleotid komplementären Polynucleotidsequenz.

**5.** Rekombinante Wirtszelle, die den Expressionsvektor enthält, der für das Polypeptid nach einem der Ansprüche 1-3 kodiert.

**Revendications**

**1.** Polypeptide isolé choisi parmi l'un des groupes constitués par :

(a) un polypeptide isolé codé par un polynucléotide présentant la séquence de SEQ ID NO:1 ;
(b) un polypeptide isolé comprenant une séquence polypeptide présentant une identité d'au moins 95% vis-à-vis de la séquence polypeptide complète de SEQ ID NO:2 ;
(c) un polypeptide isolé présentant une identité d'au moins 95% vis-à-vis de la séquence polypeptide complète de SEQ ID NO:2 ; et la séquence polypeptide de SEQ ID NO:2.

**2.** Polypeptide isolé selon la revendication 1, comprenant la séquence polypeptide de SEQ ID NO:2.

**3.** Polypeptide isolé selon la revendication 1, qui est la séquence polypeptide de SEQ ID NO:2.

**4.** Polynucléotide isolé choisi parmi l'un des groupes constitués par :

(a) un polynucléotide isolé constitué par une séquence présentant une identité d'au moins 95%, sur la totalité de la longueur, vis-à-vis du polynucléotide de SEQ ID NO:1 ;
(b) un polynucléotide isolé comprenant une séquence polynucléotide codant une séquence polypeptide présentant une identité d'au moins 95%, sur la totalité de la longueur, vis-à-vis de la séquence polypeptide de SEQ ID NO:2 ;
(c) un polynucléotide isolé qui est l'équivalent ARN d'un polynucléotide de (a) et (b) ;

ou une séquence polynucléotide qui est complémentaire vis-à-vis dudit polynucléotide isolé.

**5.** Cellule hôte recombinante comprenant le vecteur d'expression codant le polypeptide selon l'une quelconque des revendications 1 à 3.

Figure 1

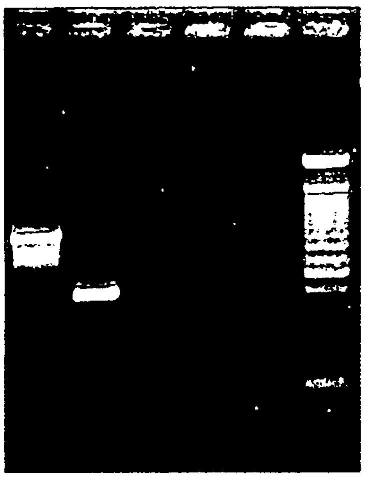

**Figure 2**

1 2 3 4 5 6 7 8 9 10 11 12 13 14 15 16 17 18 19 20

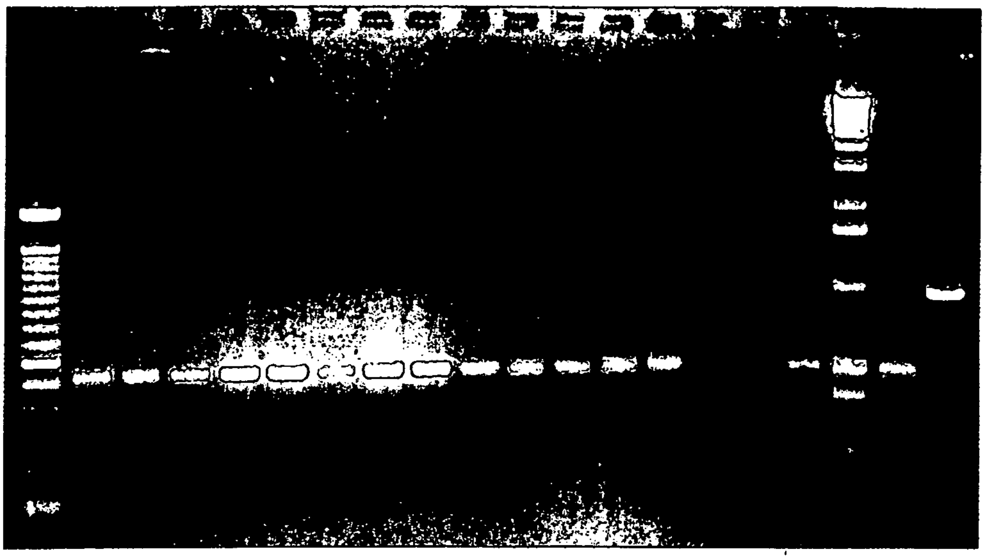

Figure 3

1 2 3 4 5 6 7 8 9 10 11 12 13 14 15

**SEQUENCE LISTING**

<110> Merck Patent GmbH

<120> Novel small GTPase

<130> MFQ-111ERws

<140>
<141>

<160> 6

<170> Patent In Ver. 2.1

<210> 1
<211> 2586
<212> DNA
<213> Homo sapiens

<220>
<221> CDS
<222> (1)..(2586)

<400> 1

```
atg cat tgt gga tta ctt cat att gac cag gac att gtc aat aca atc    48
Met His Cys Gly Leu Leu His Ile Asp Gln Asp Ile Val Asn Thr Ile
 1               5                  10                  15

atc aaa cac tgc tca cct caa ttt ttt tca ctt ggt ttg cct ggt gcc    96
Ile Lys His Cys Ser Pro Gln Phe Phe Ser Leu Gly Leu Pro Gly Ala
            20                  25                  30

aca atg ctt att atg gat ttt att gta gca gct ggt aga gtg gct tct    144
Thr Met Leu Ile Met Asp Phe Ile Val Ala Ala Gly Arg Val Ala Ser
        35                  40                  45

tca gct ttt ctc aat gca cca aga gtg gaa gca caa gtt ctt ctg gga    192
Ser Ala Phe Leu Asn Ala Pro Arg Val Glu Ala Gln Val Leu Leu Gly
    50                  55                  60

tct ttg gtt tgc ttt ccc aac tta tat tgt gaa ctg cct tct ctt cat    240
Ser Leu Val Cys Phe Pro Asn Leu Tyr Cys Glu Leu Pro Ser Leu His
65                  70                  75                  80

ccc aac att cct gat gtt gct gtg tct cag ttt aca gat gtt aag gaa    288
Pro Asn Ile Pro Asp Val Ala Val Ser Gln Phe Thr Asp Val Lys Glu
                85                  90                  95

ctt ata atc aaa act gta tta agc tcg gca aga gat gag ccc tct ggt    336
Leu Ile Ile Lys Thr Val Leu Ser Ser Ala Arg Asp Glu Pro Ser Gly
            100                 105                 110

cct gca cga tgt gta gca ctt tgt agt tta ggt att tgg att tgt gaa    384
Pro Ala Arg Cys Val Ala Leu Cys Ser Leu Gly Ile Trp Ile Cys Glu
        115                 120                 125

gaa cta gtc cat gag tct cat cat cct caa att aag gaa gct ctg aat    432
Glu Leu Val His Glu Ser His His Pro Gln Ile Lys Glu Ala Leu Asn
    130                 135                 140
```

```
gtg att tgt gtt tcc tta aag ttt act aat aaa aca gta gcc cac gta    480
Val Ile Cys Val Ser Leu Lys Phe Thr Asn Lys Thr Val Ala His Val
145                 150                 155                 160

gct tgt aac atg ctt cac atg ctg gtt cat tat gta cct aga ctt cag    528
Ala Cys Asn Met Leu His Met Leu Val His Tyr Val Pro Arg Leu Gln
            165                 170                 175

att tac cag cct gat tct ccc ttg aaa att att cag atc cta ata gct    576
Ile Tyr Gln Pro Asp Ser Pro Leu Lys Ile Ile Gln Ile Leu Ile Ala
        180                 185                 190

acc atc acc cat ctt tta cca agt aca gag gct tca tct tat gaa atg    624
Thr Ile Thr His Leu Leu Pro Ser Thr Glu Ala Ser Ser Tyr Glu Met
    195                 200                 205

gac aag agg ttg gta gta tct tta ctt ctc tgc ctt ctg gac tgg atc    672
Asp Lys Arg Leu Val Val Ser Leu Leu Leu Cys Leu Leu Asp Trp Ile
    210                 215                 220

atg gcc tta cct cta aag aca ctg ctc caa cca ttt cat gct acg gga    720
Met Ala Leu Pro Leu Lys Thr Leu Leu Gln Pro Phe His Ala Thr Gly
225                 230                 235                 240

gca gaa agc gat aaa aca gaa aaa tct gtt ctc aat tgc att tat aag    768
Ala Glu Ser Asp Lys Thr Glu Lys Ser Val Leu Asn Cys Ile Tyr Lys
            245                 250                 255

gtt tta cat ggg tgt gtt tat gga gct cag tgt ttt agc aat cca agg    816
Val Leu His Gly Cys Val Tyr Gly Ala Gln Cys Phe Ser Asn Pro Arg
        260                 265                 270

tat ttt ccc atg agc ctc tct gat ttg gca tct gta gat tat gat cct    864
Tyr Phe Pro Met Ser Leu Ser Asp Leu Ala Ser Val Asp Tyr Asp Pro
    275                 280                 285

ttt atg cat ttg gaa agt ctg aaa gag cct gag cct ctg cac tct cct    912
Phe Met His Leu Glu Ser Leu Lys Glu Pro Glu Pro Leu His Ser Pro
    290                 295                 300

gac tca gaa cga tct tct aaa ctc cag cca gta aca gaa gtg aaa act    960
Asp Ser Glu Arg Ser Ser Lys Leu Gln Pro Val Thr Glu Val Lys Thr
305                 310                 315                 320

caa atg cag cat gga tta atc tct ata gca gcc cgc act gtt att aca    1008
Gln Met Gln His Gly Leu Ile Ser Ile Ala Ala Arg Thr Val Ile Thr
            325                 330                 335

cat ctg gta aat cac ctg ggc cat tat cca atg agc ggt ggt cct gct    1056
His Leu Val Asn His Leu Gly His Tyr Pro Met Ser Gly Gly Pro Ala
        340                 345                 350

atg cta aca agt cag gtg tgt gaa aat cac gac aat cat tac agt gaa    1104
Met Leu Thr Ser Gln Val Cys Glu Asn His Asp Asn His Tyr Ser Glu
    355                 360                 365

agt act gaa ctt tct cct gaa ctc ttt gag agt cca aat atc cag ttc    1152
Ser Thr Glu Leu Ser Pro Glu Leu Phe Glu Ser Pro Asn Ile Gln Phe
    370                 375                 380

ttt gtg tta aat aat aca acc tta gtg tcc tgt atc cag atc aga tca    1200
```

```
Phe Val Leu Asn Asn Thr Thr Leu Val Ser Cys Ile Gln Ile Arg Ser
385                 390                 395                 400

gaa gag aat atg cct gga gga ggt tta tct gct ggc ctt gca tca gcc    1248
Glu Glu Asn Met Pro Gly Gly Gly Leu Ser Ala Gly Leu Ala Ser Ala
                405                 410                 415

aat tca aat gtc aga atc ata gta cgt gat ctc tct gga aaa tat tca    1296
Asn Ser Asn Val Arg Ile Ile Val Arg Asp Leu Ser Gly Lys Tyr Ser
            420                 425                 430

tgg gat tct gct ata ctg tat ggc cca cct cct gta agt ggc ttg tca    1344
Trp Asp Ser Ala Ile Leu Tyr Gly Pro Pro Pro Val Ser Gly Leu Ser
        435                 440                 445

gaa cct aca tct ttc atg ctt tca ttg tct cac caa gag aag cca gaa    1392
Glu Pro Thr Ser Phe Met Leu Ser Leu Ser His Gln Glu Lys Pro Glu
    450                 455                 460

gag cct ccg aca tct aat gaa tgc tta gaa gat ata acc gta aaa gat    1440
Glu Pro Pro Thr Ser Asn Glu Cys Leu Glu Asp Ile Thr Val Lys Asp
465                 470                 475                 480

gga ctt tct ctc cag ttt aaa aga ttt aga gaa act gta cca act tgg    1488
Gly Leu Ser Leu Gln Phe Lys Arg Phe Arg Glu Thr Val Pro Thr Trp
                485                 490                 495

gat aca ata aga gat gaa gaa gat gtt ctt gat gag ctc ttg cag tat    1536
Asp Thr Ile Arg Asp Glu Glu Asp Val Leu Asp Glu Leu Leu Gln Tyr
            500                 505                 510

ttg ggt gtt act agt cct gaa tgc tta cag aga act gga atc tca ctt    1584
Leu Gly Val Thr Ser Pro Glu Cys Leu Gln Arg Thr Gly Ile Ser Leu
        515                 520                 525

aat att cct gct cca caa cct gtg tgc att tct gaa aaa caa gaa aat    1632
Asn Ile Pro Ala Pro Gln Pro Val Cys Ile Ser Glu Lys Gln Glu Asn
    530                 535                 540

gat gtt att aat gct atc ctt aag caa cat aca gaa gaa aaa gaa ttt    1680
Asp Val Ile Asn Ala Ile Leu Lys Gln His Thr Glu Glu Lys Glu Phe
545                 550                 555                 560

gtt gag aag cac ttt aat gac tta aac atg aaa gct gtg gaa caa gat    1728
Val Glu Lys His Phe Asn Asp Leu Asn Met Lys Ala Val Glu Gln Asp
                565                 570                 575

gaa cca ata cct caa aaa cct cag tca gca ttt tat tat tgc aga ttg    1776
Glu Pro Ile Pro Gln Lys Pro Gln Ser Ala Phe Tyr Tyr Cys Arg Leu
            580                 585                 590

ctt ctt agt ata ttg gga atg aat tcc tgg gac aaa cgg agg agc ttt    1824
Leu Leu Ser Ile Leu Gly Met Asn Ser Trp Asp Lys Arg Arg Ser Phe
        595                 600                 605

cat ctc ctg aag aaa aat gaa aag cta ctt aga gaa ctt agg aac ttg    1872
His Leu Leu Lys Lys Asn Glu Lys Leu Leu Arg Glu Leu Arg Asn Leu
    610                 615                 620

gat tca agg cag tgc cga gag aca cac aag att gca gta ttt tat gtt    1920
Asp Ser Arg Gln Cys Arg Glu Thr His Lys Ile Ala Val Phe Tyr Val
```

```
625                 630                 635                 640

gct gaa gga caa gaa gac aaa cac tcc att ctc acc aat aca gga gga     1968
Ala Glu Gly Gln Glu Asp Lys His Ser Ile Leu Thr Asn Thr Gly Gly
                645                 650                 655

agt caa gca tat gaa gat ttt gta gct ggt ctt ggt tgg gag gta aat     2016
Ser Gln Ala Tyr Glu Asp Phe Val Ala Gly Leu Gly Trp Glu Val Asn
            660                 665                 670

ctt aca aac cat tgt ggt ttt atg gga gga cta caa aaa aac aaa agc     2064
Leu Thr Asn His Cys Gly Phe Met Gly Gly Leu Gln Lys Asn Lys Ser
        675                 680                 685

act gga ttg acc act cca tat ttt gct acc tct aca gta gaa gta ata     2112
Thr Gly Leu Thr Thr Pro Tyr Phe Ala Thr Ser Thr Val Glu Val Ile
    690                 695                 700

ttt cac atg tca aca aga atg cct tct gat tct gat gat tct ttg acc     2160
Phe His Met Ser Thr Arg Met Pro Ser Asp Ser Asp Asp Ser Leu Thr
705                 710                 715                 720

aaa aaa ttg aga cat ttg gga aat gat gaa gtg cac att gtt tgg tca     2208
Lys Lys Leu Arg His Leu Gly Asn Asp Glu Val His Ile Val Trp Ser
                725                 730                 735

gag cat act aga gac tac agg aga gga att att ccc aca gaa ttt ggt     2256
Glu His Thr Arg Asp Tyr Arg Arg Gly Ile Ile Pro Thr Glu Phe Gly
            740                 745                 750

gat gtc ctt att gta ata tat cca atg aaa aat cac atg ttc agt att     2304
Asp Val Leu Ile Val Ile Tyr Pro Met Lys Asn His Met Phe Ser Ile
        755                 760                 765

cag ata atg aga aaa cca gag gtt ccc ttc ttt ggt ccc ctt ttt gat     2352
Gln Ile Met Arg Lys Pro Glu Val Pro Phe Phe Gly Pro Leu Phe Asp
    770                 775                 780

ggt gct att gtg aat gga aag gtt cta ccc att atg gtt aga gca aca     2400
Gly Ala Ile Val Asn Gly Lys Val Leu Pro Ile Met Val Arg Ala Thr
785                 790                 795                 800

gct ata aat gca agc cgt gct ctg aaa tct ctg att cca ttg tat caa     2448
Ala Ile Asn Ala Ser Arg Ala Leu Lys Ser Leu Ile Pro Leu Tyr Gln
                805                 810                 815

aac ttc tat gag gag aga gca cga tac ctg caa aca att gtc cag cac     2496
Asn Phe Tyr Glu Glu Arg Ala Arg Tyr Leu Gln Thr Ile Val Gln His
            820                 825                 830

cac tta gaa cca aca aca ttt gaa gat ttt gca gca cag gtt ttt tct     2544
His Leu Glu Pro Thr Thr Phe Glu Asp Phe Ala Ala Gln Val Phe Ser
        835                 840                 845

cca gct ccc tac cac cat tta cca tct ggt gcc gat cat taa             2586
Pro Ala Pro Tyr His His Leu Pro Ser Gly Ala Asp His
    850                 855                 860
```

<210> 2

<211> 861
<212> PRT
<213> Homo sapiens

<400> 2

```
Met His Cys Gly Leu Leu His Ile Asp Gln Asp Ile Val Asn Thr Ile
 1               5                  10                  15
Ile Lys His Cys Ser Pro Gln Phe Phe Ser Leu Gly Leu Pro Gly Ala
            20                  25                  30
Thr Met Leu Ile Met Asp Phe Ile Val Ala Ala Gly Arg Val Ala Ser
        35                  40                  45
Ser Ala Phe Leu Asn Ala Pro Arg Val Glu Ala Gln Val Leu Leu Gly
    50                  55                  60
Ser Leu Val Cys Phe Pro Asn Leu Tyr Cys Glu Leu Pro Ser Leu His
65                  70                  75                  80
Pro Asn Ile Pro Asp Val Ala Val Ser Gln Phe Thr Asp Val Lys Glu
                85                  90                  95
Leu Ile Ile Lys Thr Val Leu Ser Ser Ala Arg Asp Glu Pro Ser Gly
            100                 105                 110
Pro Ala Arg Cys Val Ala Leu Cys Ser Leu Gly Ile Trp Ile Cys Glu
        115                 120                 125
Glu Leu Val His Glu Ser His His Pro Gln Ile Lys Glu Ala Leu Asn
    130                 135                 140
Val Ile Cys Val Ser Leu Lys Phe Thr Asn Lys Thr Val Ala His Val
145                 150                 155                 160
Ala Cys Asn Met Leu His Met Leu Val His Tyr Val Pro Arg Leu Gln
                165                 170                 175
Ile Tyr Gln Pro Asp Ser Pro Leu Lys Ile Ile Gln Ile Leu Ile Ala
            180                 185                 190
Thr Ile Thr His Leu Leu Pro Ser Thr Glu Ala Ser Ser Tyr Glu Met
        195                 200                 205
Asp Lys Arg Leu Val Val Ser Leu Leu Leu Cys Leu Leu Asp Trp Ile
    210                 215                 220
Met Ala Leu Pro Leu Lys Thr Leu Leu Gln Pro Phe His Ala Thr Gly
225                 230                 235                 240
Ala Glu Ser Asp Lys Thr Glu Lys Ser Val Leu Asn Cys Ile Tyr Lys
                245                 250                 255
Val Leu His Gly Cys Val Tyr Gly Ala Gln Cys Phe Ser Asn Pro Arg
            260                 265                 270
Tyr Phe Pro Met Ser Leu Ser Asp Leu Ala Ser Val Asp Tyr Asp Pro
        275                 280                 285
Phe Met His Leu Glu Ser Leu Lys Glu Pro Glu Pro Leu His Ser Pro
    290                 295                 300
Asp Ser Glu Arg Ser Ser Lys Leu Gln Pro Val Thr Glu Val Lys Thr
305                 310                 315                 320
Gln Met Gln His Gly Leu Ile Ser Ile Ala Ala Arg Thr Val Ile Thr
                325                 330                 335
His Leu Val Asn His Leu Gly His Tyr Pro Met Ser Gly Gly Pro Ala
            340                 345                 350
Met Leu Thr Ser Gln Val Cys Glu Asn His Asp Asn His Tyr Ser Glu
        355                 360                 365
Ser Thr Glu Leu Ser Pro Glu Leu Phe Glu Ser Pro Asn Ile Gln Phe
    370                 375                 380
Phe Val Leu Asn Asn Thr Thr Leu Val Ser Cys Ile Gln Ile Arg Ser
385                 390                 395                 400
Glu Glu Asn Met Pro Gly Gly Gly Leu Ser Ala Gly Leu Ala Ser Ala
                405                 410                 415
Asn Ser Asn Val Arg Ile Ile Val Arg Asp Leu Ser Gly Lys Tyr Ser
            420                 425                 430
Trp Asp Ser Ala Ile Leu Tyr Gly Pro Pro Pro Val Ser Gly Leu Ser
        435                 440                 445
Glu Pro Thr Ser Phe Met Leu Ser Leu Ser His Gln Glu Lys Pro Glu
```

```
        450                 455                 460
Glu Pro Pro Thr Ser Asn Glu Cys Leu Glu Asp Ile Thr Val Lys Asp
465                 470                 475                 480
Gly Leu Ser Leu Gln Phe Lys Arg Phe Arg Glu Thr Val Pro Thr Trp
                485                 490                 495
Asp Thr Ile Arg Asp Glu Glu Asp Val Leu Asp Glu Leu Leu Gln Tyr
            500                 505                 510
Leu Gly Val Thr Ser Pro Glu Cys Leu Gln Arg Thr Gly Ile Ser Leu
        515                 520                 525
Asn Ile Pro Ala Pro Gln Pro Val Cys Ile Ser Glu Lys Gln Glu Asn
    530                 535                 540
Asp Val Ile Asn Ala Ile Leu Lys Gln His Thr Glu Glu Lys Glu Phe
545                 550                 555                 560
Val Glu Lys His Phe Asn Asp Leu Asn Met Lys Ala Val Glu Gln Asp
                565                 570                 575
Glu Pro Ile Pro Gln Lys Pro Gln Ser Ala Phe Tyr Tyr Cys Arg Leu
            580                 585                 590
Leu Leu Ser Ile Leu Gly Met Asn Ser Trp Asp Lys Arg Arg Ser Phe
        595                 600                 605
His Leu Leu Lys Lys Asn Glu Lys Leu Leu Arg Glu Leu Arg Asn Leu
    610                 615                 620
Asp Ser Arg Gln Cys Arg Glu Thr His Lys Ile Ala Val Phe Tyr Val
625                 630                 635                 640
Ala Glu Gly Gln Glu Asp Lys His Ser Ile Leu Thr Asn Thr Gly Gly
                645                 650                 655
Ser Gln Ala Tyr Glu Asp Phe Val Ala Gly Leu Gly Trp Glu Val Asn
            660                 665                 670
Leu Thr Asn His Cys Gly Phe Met Gly Gly Leu Gln Lys Asn Lys Ser
        675                 680                 685
Thr Gly Leu Thr Thr Pro Tyr Phe Ala Thr Ser Thr Val Glu Val Ile
    690                 695                 700
Phe His Met Ser Thr Arg Met Pro Ser Asp Ser Asp Asp Ser Leu Thr
705                 710                 715                 720
Lys Lys Leu Arg His Leu Gly Asn Asp Glu Val His Ile Val Trp Ser
                725                 730                 735
Glu His Thr Arg Asp Tyr Arg Arg Gly Ile Ile Pro Thr Glu Phe Gly
            740                 745                 750
Asp Val Leu Ile Val Ile Tyr Pro Met Lys Asn His Met Phe Ser Ile
        755                 760                 765
Gln Ile Met Arg Lys Pro Glu Val Pro Phe Phe Gly Pro Leu Phe Asp
    770                 775                 780
Gly Ala Ile Val Asn Gly Lys Val Leu Pro Ile Met Val Arg Ala Thr
785                 790                 795                 800
Ala Ile Asn Ala Ser Arg Ala Leu Lys Ser Leu Ile Pro Leu Tyr Gln
                805                 810                 815
Asn Phe Tyr Glu Glu Arg Ala Arg Tyr Leu Gln Thr Ile Val Gln His
            820                 825                 830
His Leu Glu Pro Thr Thr Phe Glu Asp Phe Ala Ala Gln Val Phe Ser
        835                 840                 845
Pro Ala Pro Tyr His His Leu Pro Ser Gly Ala Asp His
    850                 855                 860
```

<210> 3
<211> 23
<212> DNA
<213> Artificial Sequence

<220>
<223> Description of Artificial Sequence: Primer01

<400> 3
tacgggagca gaaagcgata aaa 23

<210> 4
<211> 24
<212> DNA
<213> Artificial Sequence

<220>
<223> Description of Artificial Sequence: Primer02

<400> 4
gcccagcaga taaacctcct ccag 24

<210> 5
<211> 26
<212> DNA
<213> Artificial Sequence

<220>
<223> Description of Artificial Sequence: Primer03

<400> 5
tgaaggtcgg agtcaacgga tttggt 25

<210> 6
<211> 24
<212> DNA
<213> Artificial Sequence

<220>
<223> Description of Artificial Sequence: Primer04

<400> 6
catgtgggcc atgaggtcca ccac 24